(19) Europäisches Patentamt
European Patent Office
Office européen des brevets

(11) Veröffentlichungsnummer: **0 019 247**
**B1**

(12) **EUROPÄISCHE PATENTSCHRIFT**

(45) Veröffentlichungstag der Patentschrift: **25.11.81**

(51) Int. Cl.³: **C 07 J 1/00**, C 07 J 17/00, A 61 K 31/565 // C07J41/00

(21) Anmeldenummer: **80102599.0**

(22) Anmeldetag: **10.05.80**

(54) **16-Alpha-Alkylsteroide, Verfahren zu ihrer Herstellung und diese enthaltende pharmazeutische Präparate.**

(30) Priorität: **17.05.79 DE 2920184**

(43) Veröffentlichungstag der Anmeldung: **26.11.80 Patentblatt 80/24**

(45) Bekanntmachung des Hinweises auf die Patenterteilung: **25.11.81 Patentblatt 81/47**

(84) Benannte Vertragsstaaten: **AT BE CH DE FR GB IT LI LU NL SE**

(56) Entgegenhaltungen:
**DE-A-1 814 304**
**DE-A-2 037 403**
**FR-M-5 260**

(73) Patentinhaber: **SCHERING AKTIENGESELLSCHAFT Berlin und Bergkamen, Müllerstrasse 170/178 Postfach 65 0311, D-1000 Berlin 65 (DE)**

(72) Erfinder: **Neef, Günter, Dr., Darmstädter Strasse 9, D-1000 Berlin 15 (DE)**
Erfinder: **Eder, Ulrich, Dr., Zeltingerstrasse 17, D-1000 Berlin 28 (DE)**
Erfinder: **Haffer, Gregor, Dr., Alt Mariendorf 36, D-1000 Berlin 42 (DE)**
Erfinder: **Sauer, Gerhard, Dr., Königsbacher Zeile 41 A, D-1000 Berlin 28 (DE)**
Erfinder: **Wiechert, Rudolf, Prof. Dr., Petzower Strasse 8 A, D-1000 Berlin 39 (DE)**
Erfinder: **Steinbeck, Hermann, Dr., Hammerstrasse 46, D-1000 Berlin 37 (DE)**

**EP 0 019 247 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# 16-Alpha-Alkylsteroide, Verfahren zu ihrer Herstellung und diese enthaltende pharmazeutische Präparate

Die Erfindung betrifft 16α-Alkylsteroide der allgemeinen Formel I

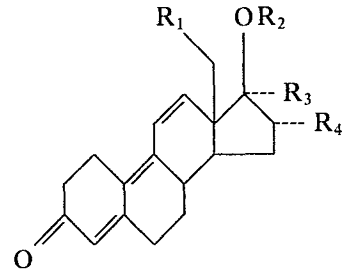

(I)

worin

$R_1$ ein Wasserstoffatom oder eine Methylgruppe,
$R_2$ ein Wasserstoffatom, eine Alkyl-, Acyl- oder Glykosylgruppe,
$R_3$ ein Wasserstoffatom oder einen substituierten oder unsubstituierten, gesättigten oder ungesättigten niederen Kohlenwasserstoffrest und
$R_4$ eine Methyl-, Ethyl- oder Propylgruppe

bedeuten.

Die Erfindung betrifft weiterhin ein Verfahren zur Herstellung der 16α-Alkylsteroide der allgemeinen Formel I, das dadurch gekennzeichnet ist, daß man ein 16α-Alkylsteroid der allgemeinen Formel II

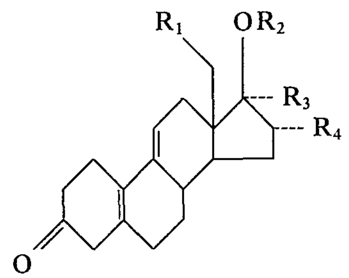

(II)

in der $R_1$, $R_2$, $R_3$ und $R_4$ die angegebene Bedeutung haben, dehydriert und gegebenenfalls anschließend eine veresterte sekundäre 17β-Hydroxygruppe verseift und/oder eine freie sekundäre oder tertiäre 17β-Hydroxygruppe verestert, glykosidiert oder veräthert.

Die Verbindungen der allgemeinen Formel I besitzen wertvolle pharmakologische Eigenschaften. So zeigen bereits die Verbindungen ohne einen Substituenten in 17α-Stellung ($R_3$=H) eine dem Levonorgestrel entsprechende gestagene Aktivität und nur eine sehr geringe androgene Wirkung. Die Eigenschaften der neuen 16α-Alkylsteroide der allgemeinen Formel I waren nicht vorauszusehen; denn die entsprechenden Verbindungen ohne einen 16α-Alkylsubstituenten zeigen sehr starke androgene Wirksamkeit (Comptes Rendus Acad. Sc. 257 (1963), 569–570).

Aufgrund ihrer günstigen pharmakologischen Eigenschaften können die erfindungsgemäßen Verbindungen besonders vorteilhaft in Antikonzeptionspräparaten Verwendung finden, wobei sie als Gestagenkomponente in Kombination mit einer östrogen wirksamen Hormonkomponente, wie zum Beispiel Äthinylöstradiol, oder als alleinige Wirkkomponente eingesetzt werden. Die Verbindungen können aber auch in Präparaten zur Behandlung gynäkologischer Störungen eingesetzt werden.

Zur Anwendung werden die neuen Verbindungen mit den in der galenischen Pharmazie üblichen Zusätzen, Trägersubstanzen und Geschmackskorrigentien nach an sich bekannten Methoden zu den üblichen Arzneimittelformen verarbeitet. Für die orale Applikation kommen insbesondere Tabletten, Dragées, Kapseln, Pillen, Suspensionen oder Lösungen in Frage. Für die parenterale Applikation kommen insbesondere ölige Lösungen, wie zum Beispiel Sesamöl- oder Rizinusöllösungen in Frage, die gegebenenfalls zusätzlich noch ein Verdünnungsmittel, wie zum Beispiel Benzylbenzoat oder Benzylalkohol, enthalten können. Die Konzentration des Wirkstoffes ist abhängig von der Applikationsform. So enthalten beispielsweise Tabletten zur oralen Applikation vorzugsweise 0,01–0,5 mg Wirkstoff und Lösungen zur parenteralen Applikation vorzugsweise 1–100 mg Wirkstoff pro 1 ml Lösung.

Die Dosierung der erfindungsgemäßen pharmazeutischen Präparate kann sich mit der Form und dem Zweck der Verabfolgung ändern. Beispielsweise liegt die tägliche kontrazeptive Dosis bei oraler Applikation bei 0,05 bis 0,5 mg Wirkstoff gemäß Formel I.

Die Steroide der allgemeinen Formel I gemäß Anspruch 1 können in 17α-Stellung unsubstituiert sein

($R_3$=H) oder als Substituenten $R_3$ einen substituierten oder unsubstituierten gesättigten oder ungesättigten niederen Kohlenwasserstoffrest tragen. Solche niederen Kohlenwasserstoffreste sind zum Beispiel Alkylreste mit 1–5 Kohlenstoffatomen, vorzugsweise der Methyl- und Ethylrest, Alkenyl- und Alkinylreste mit 2–3 Kohlenstoffatomen, vorzugsweise der Vinyl-, Ethinyl- und Propinylrest. Der Kohlenwasserstoffrest kann auch in geeigneter Weise durch eine oder mehrere Hydroxygruppen oder Halogenatome substituiert sein, wobei der Chlorethinylrest als substituierter Kohlenwasserstoffrest bevorzugt ist.

Alkylgruppen $R_2$ können 1–5 Kohlenstoffatome enthalten und gegebenenfalls durch ein Sauerstoffatom unterbrochen sein, bevorzugt sind die Methyl-, die Ethyl-, die Methoxymethyl-, Methoxyethyl-, die Ethoxyethyl- und die Tetrahydropyranylgruppe.

Als Acylgruppen $R_2$ kommen solche von physiologisch verträglichen Säuren in Frage. Bevorzugt sind solche, die sich von organischen Carbon- oder Sulfonsäuren mit 1–17 Kohlenstoffatomen ableiten. Carbon- und Sulfonsäuren können der aliphatischen, cycloaliphatischen, aromatischen, aromatischaliphatischen oder heterocyclischen Reihe angehören. Diese Säuren können darüber hinaus noch gesättigt oder ungesättigt, ein- oder mehrbasisch und/oder in üblicher Weise substituiert sein. Als Beispiel für die Substituenten seien Hydroxy-, Alkoxy-, Acyloxy-, Oxo-, Aminogruppen oder Halogenatome genannt.

Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diäthylessigsäure, tert.-Butylessigsäure, $\beta$-Cyclopentylpropionsäure, Cyclohexylessigsäure, Cyclohexancarbonsäure, Phenylessigsäure, Phenoxyessigsäure, Mono-, Di- und Trichloressigsäure, Aminoessigsäure, Diäthylaminoessigsäure, Piperidinoessigsäure, Morpholinoessigsäure, Milchsäure, Bernsteinsäure, Adipinsäure, Benzoesäure, Nikotinsäure, Isonikotinsäure, Furan-2-carbonsäure, O-Tridecanoylglykolsäure, O-Hexadecanoylglykolsäure, $\beta$-Tridecanoyloxypropionsäure. Als Sulfonsäuren seien genannt: Methan-, Äthan-, $\beta$-Chloräthan-, Propan-, Isopropan-, Butan-, Cyclopentan-, Cyclohexan-, Benzol-, p-Toluol-, p-Chlorbenzolsulfonsäure, ferner N,N-Dimethyl-, N,N-Diäthyl-, Bis($\beta$-Chloräthyl)-aminosulfonsäure, Pyrrolidino-, Piperidino-, Piperazino-, N-Methylpiperazino- und Morpholinosulfonsäure.

Ganz besonders bevorzugt sind Alkancarbonsäuren mit 2–7 Kohlenstoffatomen.

Der Ausdruck »Glykosyl« soll freie oder mit niederen Alkancarbonsäuren veresterte Zuckerreste bedeuten. Niedere Alkancarbonsäuren können bis zu 5 Kohlenstoffatome enthalten. Als Zucker seien beispielsweise Glucose, Rhamnose, Ribose und Arabinose genannt.

Die Dehydrierung einer Verbindung der allgemeinen Formel II gemäß Verfahrensanspruch kann in an sich bekannter Weise mit Dehydrierungsmitteln vorgenommen werden. Geeignete Dehydrierungsmittel sind beispielsweise Selendioxid und substituierte p-Chinone, insbesondere substituierte p-Benzochinone, wie 2,3-Dichlor-5,6-dicyan-benzochinon (DDQ), 2,3-Dibrom-5,6-dicyanbenzochinon, 2,3-Dicyan-4-chlor-benzochinon, 2,3-Dicyanbenzochinon und 2,3,5,6-Tetrachlor-benzochinon (Chloranil).

Die Dehydrierung wird zweckmäßigerweise in einem organischen Lösungsmittel vorgenommen, wie zum Beispiel mit substituierten Chinonen in Methylenchlorid, Dichlorethan, Benzol, Toluol, Dioxan, Essigester und auch in Chlorbenzol, Diethylether, Ethylenglykol, Dimethylformamid, Nitrobenzol, Dimethylsulfoxid u. a. Die Reaktion kann bei Raumtemperatur oder unter erhöhter Temperatur durchgeführt werden; vorzugsweise wird bei Raumtemperatur unter Inertgas gearbeitet. Die Dehydrierung von $\Delta^{5(10),9(11)}$-Steroiden mit substituierten Chinonen zu $\Delta^{4,9,11}$-Steroiden wird bereits in der US-Patentschrift 3 453 267 ausführlich beschrieben.

Die Dehydrierung mit Selendioxid erfolgt beispielsweise in Dioxan in der Siedehitze.

Die Verseifung von 17$\beta$-Acyloxygruppen kann in an sich bekannter Weise erfolgen. Beispielsweise wird die Verseifung mit Basen in wäßrig-alkoholischer Lösung, wie mit Kaliumcarbonat in wäßrig-methanolischer Lösung, vorgenommen.

Für die sich gegebenenfalls anschließende Veresterung der sekundären oder tertiären Hydroxygruppe kommen die üblicherweise in der Steroidchemie zur Veresterung angewendeten Verfahren in Frage. Beispielsweise sei die Umsetzung mit Säuren oder Säureanhydriden in Gegenwart starker Säuren, wie zum Beispiel Trifluoressigsäure oder p-Toluolsulfonsäure, bei Raumtemperatur oder etwas angehobener Temperatur, oder die Umsetzung mit einem Säureanhydrid in Gegenwart eines tertiären Amins in der Wärme bei etwa 20–200° C genannt.

Werden Pyridin und 4-(Dimethylamino)-pyridin als tertiäre Amine gemeinsam angewandt, kann die Veresterung bei Raumtemperatur durchgeführt werden.

Zur Veretherung dienen alkylierende Verbindungen, wie zum Beispiel Alkylhalogenide. Die Veretherung erfolgt in an sich bekannter Weise in Gegenwart einer starken Base, wie Natronlauge, unter Verwendung eines polaren Lösungsmittels, wie Hexamethylphosphorsäuretriamid, bei 0–50° C oder in Gegenwart einer starken Base, wie Natriumhydrid, unter Verwendung eines Ethers, wie Tetrahydrofuran, bei 30–100° C.

Zur Herstellung von Alkylethern, deren Kohlenstoffkette durch ein Sauerstoffatom unterbrochen und gegebenenfalls ringgeschlossen ist, werden die 17-Hydroxyverbindungen mit Dihydropyran oder

Alkylvinylethern in Gegenwart einer starken Säure, wie p-Toluolsulfonsäure oder Phosphoroxychlorid, in die entsprechenden Tetrahydropyranyl- oder Alkoxyethylether überführt. Die Reaktion wird vorzugsweise in Gegenwart von inerten Lösungsmitteln, wie Chloroform, Methylenchlorid, Tetrahydrofuran und Dioxan, bei einer Reaktionstemperatur von −20 bis 100°C durchgeführt. Zur Herstellung von Methoxymethylethern wird die 17-Hydroxyverbindung zum Beispiel mit Formaldehyddimethylacetal in wasserfreiem Methylenchlorid in Gegenwart von Phosphorpentoxid bei Raumtemperatur umgesetzt.

Zur Glykosidierung werden die 17-Hydroxyverbindungen mit den 1-Halogenzuckern umgesetzt. Die Umsetzung erfolgt in Gegenwart eines Schwermetallsalzes oder eines Schwermetalloxids, vorzugsweise in einem inerten organischen Lösungsmittel, wie Benzol, und vorzugsweise bei der Siedetemperatur des Lösungsmittels. Als Schwermetallverbindungen werden vorzugsweise die Oxide, Cyanide, Carbonate oder Perchlorate des Silbers oder Quecksilbers verwendet.

Die als Ausgangsmaterialien verwendeten Verbindungen der allgemeinen Formel II können nach an sich bekannten Methoden hergestellt werden.

Zur Herstellung von Verbindungen der allgemeinen Formel II mit $R_1$ in der Bedeutung von Wasserstoff wird Östronmethylether zunächst gemäß deutsche Offenlegungsschrift 2 757 157 in die entsprechenden 16α-Alkyl-östronmethylether überführt und anschließend analog den in der US-Patentschrift 3 453 267 beschriebenen Verfahren durch Reduktion des 17-Ketons mit Natriumborhydrid, Birch-Reduktion des Aromatenmethylethers, saure Hydrolyse des Birch-Produkts zum $\Delta^{5(10)}$-3-Oxoderivat, Umsetzung mit Pyridinhydrobromidperbromid in Gegenwart von Pyridin zum $\Delta^{4,9(10)}$-3-Oxoderivat, Acetylierung der 17-Hydroxygruppe, Ketalisierung zum $\Delta^{5(10),9(11)}$-3-Ketal und anschließend saure Ketalspaltung in Gegenwart von Brenztraubensäure oder Oxalsäure zu 17β-Acetoxy-16α-alkyl-5(10),9(11)-estradien-3-on umgewandelt.

1. 17β-Acetoxy-16α-methyl-5(10),9(11)-estradien-3-on

a) Eine Lösung von 5 g 3-Methoxy-1,3,5(10)-estratrien-17-on in 100 ml Ethanol, 15 ml Dimethylhydrazin und 3 ml Orthoameisensäuretriethylester wird 30 Stunden unter Rückfluß erhitzt. Nach dem Abkühlen gießt man in ca. 500 ml Wasser, extrahiert mit Essigester, wäscht die Essigester-Extrakte mit gesättigter Kochsalzlösung, trocknet über Natriumsulfat und engt im Vakuum ein. Der ölige Rückstand wird aus Acetonitril kristallisiert. Man erhält 5,6 g 3-Methoxy-1,3,5(10)-estratien-17-on-N,N-dimethylhydrazon vom Schmelzpunkt 85−86°C.

b) Eine Lösung von 5 g 3-Methoxy-1,3,5(10)-estratrien-17-on-N,N-dimethylhydrazon in 50 ml absolutem Tetrahydrofuran (THF) wird bei 0°C tropfenweise mit 13 ml einer 15%igen Lösung von n-Butyllithium in Hexan versetzt. Nach Zugabe rührt man 60 Minuten unter Argon bei 0°C, tropft anschließend 1,3 ml Methyliodid bei 0°C dazu und rührt weitere 30 Minuten bei Raumtemperatur. Zur Aufarbeitung gießt man in gesättigte Ammoniumchloridlösung und extrahiert mit Essigester. Nach Kristallisation aus Acetonitril erhält man 5 g 3-Methoxy-16α-methyl-1,3,5(10)-estratrien-17-on-N,N-dimethylhydrazon vom Schmelzpunkt 122−124°C.

c) Eine Lösung von 1,4 g 3-Methoxy-16α-methyl-1,3,5(10)-estratrien-17-on-N,N-dimethylhydrazon in 60 ml Tetrahydrofuran (THF) und 12 ml Wasser wird mit einer Lösung von 1,45 g Kupfer(II)-chlorid ($CuCl_2 \cdot 2\,H_2O$) in 19 ml Wasser versetzt und 16 Stunden bei Raumtemperatur gerührt. Anschließend gießt man in Wasser und extrahiert mit Essigester. Nach Kristallisation aus Methanol erhält man 1,2 g 3-Methoxy-16α-methyl-1,3,5(10)-estratrien-17-on vom Schmelzpunkt 115−116°C.

d) 16,0 g 3-Methoxy-16α-methyl-1,3,5(10)-estratrien-17-on werden in 200 ml Ethanol gelöst und unter Eiswasserkühlung tropfenweise mit einer Lösung von 2,1 g Natriumborhydrid in 100 ml 80%igem wäßrigen Ethanol versetzt. Man rührt 16 Stunden bei Raumtemperatur, gibt danach vorsichtig 1 N-Salzsäure zur Reaktionslösung, gießt in Wasser und extrahiert mit Essigester. Nach Kristallisation aus Acetonitril erhält man 13,4 g 3-Methoxy-16α-methyl-1,3,5(10)-estratrien-17β-ol vom Schmelzpunkt 100−102°C.

e) Eine Lösung von 10,0 g 3-Methoxy-16α-methyl-1,3,5(10)-estratrien-17β-ol in 200 ml absolutem THF und 20 ml tert.-Butanol wird bei −50°C zu ca. 500 ml Ammoniak getropft und anschließend portionsweise mit 6,3 g Lithium versetzt. Man rührt 3 Stunden bei −40°C, läßt Ammoniak über Nacht verdampfen, nimmt den Rückstand in ca. 1 l Wasser auf und extrahiert mit Essigester. Man erhält 9,6 mg 3-Methoxy-16α-methyl-2,5(10)-estradien-17β-ol als amorphes Rohprodukt, das ohne weitere Reinigung in die Folgestufe eingesetzt wird.

9,6 g des obigen Produkts werden in 400 ml Aceton und 100 ml Wasser mit 7,5 g Oxalsäure 60 Minuten bei 40°C gerührt. Nach dem Abkühlen gießt man in Wasser, extrahiert mit Methylenchlorid, wäscht die Methylenchloridextrakte mit gesättigter $NaHCO_3$-Lösung und gesättigter NaCl-Lösung, trocknet über Natriumsulfat und engt ein. Man erhält 8,5 g 17β-Hydroxy-16α-methyl-5(10)-estren-3-on, das ohne weitere Reinigung in die Folgereaktion eingesetzt wird.

Eine Lösung von 8,5 g des obigen Rohproduktes in 390 ml Pyridin wird unter Eiswasserkühlung portionsweise mit 12,1 g Pyridinhydrobromid-perbromid versetzt und 2 Stunden bei 50°C unter Argon gerührt. Nach dem Abkühlen gießt man in ca. 2 l 2 N-Salzsäure und extrahiert mit Essigester. Die

Essigesterextrakte werden nacheinander mit 4 N-Salzsäure und Wasser gewaschen, über Natriumsulfat getrocknet und eingeengt. Das ölige Rohprodukt wird an Kieselgel mit Benzin/Essigester chromatographiert. Man erhält 7,8 g 17β-Hydroxy-16α-methyl-4,9(10)-estradien-3-on vom Schmelzpunkt 146–148° C (aus Essigester/Diisopropylether).

f) Eine Lösung von 7,8 g 17β-Hydroxy-16α-methyl-4,9(10)-estradien-3-on in 25 ml Pyridin und 8 ml Acetanhydrid wird 6 Stunden bei 50°C unter Argon gerührt. Nach dem Abkühlen gießt man in gesättigter $NaHCO_3$-Lösung und extrahiert mit Essigester. Die Essigesterextrakte werden nacheinander mit Wasser, 2 N-Salzsäure und gesättigter NaCl-Lösung gewaschen, über Natriumsulfat getrocknet und eingeengt. Nach Kristallisation aus Ether erhält man 6,6 g 17β-Acetoxy-16α-methyl-4,9(10)-estradien-3-on vom Schmelzpunkt 124–126° C.

g) Eine Lösung von 3,6 g 17β-Acetoxy-16α-methyl-4,9(10)-estradien-3-on in 6,5 ml Ethanol, 30 ml Orthoameisensäuretriäthylester und 100 mg p-Toluolsulfonsäure wird bei Raumtemperatur 45 Minuten gerührt. Anschließend gießt man in gesättigte $NaHCO_3$-Lösung und extrahiert mit Essigester. Nach dem Einengen erhält man 3,8 g 17β-Acetoxy-16α-methyl-5(10),9(11)-estradien-3,3-diethylketal als farbloses Öl, das ohne Reinigung in die Folgestufe eingesetzt wird.

Eine Lösung von 3,8 g des obigen Rohprodukts in 150 ml Aceton und 20 ml Wasser wird nach Zusatz von 3,0 g Oxalsäure 60 Minuten bei 40°C gerührt. Anschließend gießt man in gesättigte $NaHCO_3$-Lösung und extrahiert mit Essigester. Man erhält 3,2 g 17β-Acetoxy-16α-methyl-5(10),9(11)-estradien-3-on als farbloses Öl.

2. 17β-Acetoxy-16α-ethyl-5(10),9(11)-estradien-3-on

Gemäß 1b) werden aus 20,4 g 3-Methoxy-1,3,5(10)-estratrien-17-on-N,N-dimethylhydrazon nach Metallierung und Alkylierung mit 9,2 ml Bromethan 20,2 g 16α-Ethyl-3-methoxy-1,3,5(10)-estratrien-17-on-N,N-dimethylhydrazon vom Schmelzpunkt 101–103°C (aus Acetonitril) erhalten. Aus dem Hydrazon werden durch Umsetzung mit $CuCl_2 \cdot 2\,H_2O$ gemäß 1c) das 17-Keton und anschließend durch Reduktion des 17-Ketons mit Natriumborhydrid gemäß 1d) 16,0 g 16α-Ethyl-3-methoxy-1,3,5(10)-estratrien-17β-ol vom Schmelzpunkt 75–76° C (aus Methanol) hergestellt. 10,0 g 16α-Ethyl-3-methoxy-1,3,5(10)-estratrien-17β-ol werden analog 1e) nacheinander der Birch-Reduktion, der sauren Hydrolyse und der Umsetzung mit Pyridinhydrobromid-perbromid unterworfen. Man erhält so 7,8 g 16α-Ethyl-17β-hydroxy-4,9(10)-estradien-3-on vom Schmelzpunkt 132–135°C (aus Essigester/Diisopropylether), welches analog 1f) mit Acetatanhydrid/Pyridin in das 17-Acetat vom Schmelzpunkt 124–126°C (aus Ether) und analog 1g) durch Ketalisierung und anschließende Ketalspaltung in das 17β-Acetoxy-16α-ethyl-5(10),9(11)-estradien-3-on überführt wird.

3. 17β-Acetoxy-16α-propyl-5(10),9(11)-estradien-3-on

Gemäß 1b) werden aus 5,0 g 3-Methoxy-1,3,5(10)-estratrien-17-on-N,N-dimethylhydrazon mit 1-Brompropan nach Kristallisieren aus Acetonitril 5,4 g 3-Methoxy-16α-propyl-1,3,5(10)-estratrien-17-on-N,N-dimethylhydrazon vom Schmelzpunkt 83–85°C erhalten. Aus 5,0 g Hydrazon werden durch Umsetzung mit $CuCl_2 \cdot 2\,H_2O$ gemäß 1c) 4,1 g 3-Methoxy-16α-propyl-1,3,5(10)-estratrien-17-on vom Schmelzpunkt 93–100° C (aus Acetonitril) erhalten und aus 3,2 g des 17-Ketons mit Natriumborhydrid gemäß 1d) 2,1 g 3-Methoxy-16α-propyl-1,3,5(10)-estratrien-17β-ol vom Schmelzpunkt 54–56°C (aus Acetonitril). Durch Birch-Reduktion, saure Hydrolyse, Umsetzung mit Pyridinhydrobromidperbromid, Acetylierung in 17-Stellung, Ketalisierung und Entketalisierung in 3-Stellung wird 17β-Acetoxy-16α-propyl-5(10),9(11)-estradien-3-on als Öl erhalten.

Zur Herstellung von Verbindungen der allgemeinen Formel II mit $R_1$ in der Bedeutung von Methyl wird 18-Methyl-östronmethylether mit Bis-dimethylamino-tert.-butoxymethan in die 16-Dimethylaminomethylenverbindung überführt, deren Umsetzung mit Alkyllithium oder Alkylmagnesiumbromid führt zur 16-Alkylidenverbindung, die durch Hydrierung zur 16β-Alkylverbindung und durch saure oder alkalische Isomerisierung zum 16α-Alkyl-18-methyl-östronmethylether umgewandelt wird. Die weiteren Umsetzungen verlaufen in der gleichen Weise wie bei den Verbindungen mit $R_1$ = Wasserstoff.

4. 17β-Acetoxy-16α-ethyl-18-methyl-5(10),9(11)-estradien-3-on

a) Ein Gemisch aus 10 g 3-Methoxy-18-methyl-1,3,5(10)-estratrien-17-on und 15 g Bis-dimethylamino-tert.-butoxymethan wird 3 Stunden bei 160°C gerührt. Nach dem Abkühlen versetzt man mit 50 ml Ethanol und beläßt bei −5°C zur Kristallisation. Nach Abfiltrieren und Trocknen erhält man 10,6 g 16-Dimethylaminomethylen-3-methoxy-18-methyl-1,3,5(10)-estratrien-17-on vom Schmelzpunkt 157–159°C.

b) Eine Lösung von 5,0 g 16-Dimethylaminomethylen-3-methoxy-18-methyl-1,3,5(10)-estratrien-17-on in 150 ml Toluol wird bei −10° C tropfenweise mit 30 ml einer 5%igen Lösung von Methyllithium in Äther versetzt. Nach dem Zutropfen rührt man 15 Minuten bei −10° C, gibt anschließend ca. 10 ml Wasser zur Reaktionslösung und gießt diese dann in ca. 200 ml 0,5 N-Salzsäure. Die organische Phase wird abgetrennt, über Natriumsulfat getrocknet und eingeengt. Nach Kristallisation des Rückstandes aus Methanol erhält man 4,9 g 16-Ethyliden-3-methoxy-18-methyl-1,3,5(10)-estratrien-17-on vom Schmelzpunkt 155−157° C.

c) Eine Lösung von 1,3 g 16-Ethyliden-3-methoxy-18-methyl-1,3,5(10)-1,3,5(10)-estratrien-17-on in 180 ml Ethanol wird nach Zusatz von 130 mg 10%iger Palladiumkohle bei Raumtemperatur und Normaldruck hydriert. Nach Aufnahme von 115 ml Wasserstoff filtriert man vom Katalysator ab und engt das Filtrat ein. Kristallisation aus Methanol ergibt 1,3 g 16β-Ethyl-3-methoxy-18-methyl-I,3,5(10)-estratrien-17-on vom Schmelzpunkt 101−102° C.

d) Eine Lösung von 1,3 g 16β-Ethyl-3-methoxy-18-methyl-1,3,5(10)-estratrien-17-on in 14,5 ml Eisessig und 2 ml konzentrierte Salzsäure wird 16 Stunden bei Raumtemperatur gerührt. Anschließend gießt man in Wasser, extrahiert mit Essigester, wäscht die Essigesterextrakte nacheinander mit gesättigter $NaHCO_3$-Lösung und gesättigter NaCl-Lösung, trocknet über Natriumsulfat und engt im Vakuum ein. Das ölige Rohprodukt wird gemäß 1d) mit Natriumborhydrid reduziert. Nach Chromatographie an Kieselgel mit Benzin/Essigester erhält man 620 mg 16α-Ethyl-3-methoxy-18-methyl-1,3,5(10)-estratrien-17β-ol vom Schmelzpunkt 112−114° C (aus Methanol).

Durch Birch-Reduktion, saure Hydrolyse, Umsetzung mit Pyridinhydrobromid-perbromid, Acetylierung in 17-Stellung, Ketalisierung und Entketalisierung in 3-Stellung wird 17β-Acetoxy-16α-ethyl-18-methyl-5(10),9(11)-estradien-3-on als Öl erhalten.

5. 17α-Ethinyl-17β-hydroxy-16α-methyl-5(10),9(11)-estradien-3-on

a) Eine Lösung von 4,58 g 17β-Hydroxy-16α-methyl-4,9(10)-estradien-3-on (hergestellt nach 1e) in 200 ml Aceton wird unter Eiswasserkühlung tropfenweise mit 8,5 ml Jones-Reagenz (J. Chem. Soc. [London], 1946, 39) versetzt. Man rührt nach Zugabe 20 Minuten bei Raumtemperatur, gießt danach in Wasser und extrahiert mit Methylenchlorid. Die Methylenchloridextrakte werden mit gesättigter Kochsalzlösung gewaschen, über Natriumsulfat getrocknet und eingeengt. Man erhält nach Kristallisation aus Essigester/Diisopropylether 4,3 g 16α-Methyl-4,9(10)-estradien-3,17-dion vom Schmelzpunkt 140−143° C.

b) Eine Lösung von 4,2 g des unter a) erhaltenen Dions in 8 ml Ethanol und 38 ml Orthoameisensäuretriethylester wird nach Zusatz von 100 mg p-Toluolsulfonsäure 45 Minuten bei Raumtemperatur gerührt. Anschließend gießt man in gesättigte $NaHCO_3$-Lösung und extrahiert mit Essigester. Man erhält 4,4 g 3,3-Diethoxy-16α-methyl-5(10),9(11)-estradien-17-on als farbloses Öl, das ohne weitere Reinigung in die Folgestufe eingesetzt wird.

c) Unter Eiswasserkühlung leitet man über 30 Minuten einen Acetylenstrom durch 140 ml absoluten Tetrahydrofuran (THF). Anschließend tropft man bei 0° C 36 ml einer 15%igen Lösung von n-Butyllithium in Hexan hinzu und leitet erneut Acetylen durch die Lösung (10 Minuten). Unter Beibehaltung der Temperatur (0° C) tropft man eine Lösung von 4,1 g des unter b) erhaltenen Ketals in 40 ml THF hinzu und rührt 60 Minuten bei 0° C. Zur Aufarbeitung gießt man in gesättigte $NH_4Cl$-Lösung und extrahiert mit Essigester.

Das ölige Rohprodukt wird in 180 ml Aceton und 40 ml Wasser aufgenommen und nach Zusatz von 3,4 g Oxalsäure 60 Minuten bei 40° C gerührt. Man gießt danach in Wasser und extrahiert mit Essigester. Nach Chromatographie an Kieselgel mit Benzin/Aceton erhält man 2,6 g 17α-Ethinyl-17β-hydroxy-16α-methyl-5(10),9(11)-estradien-3-on vom Schmelzpunkt 165−168° C (Essigester/Ether).

Analog erhält man aus 16α-Ethyl-17β-hydroxy-4,9(10)-estradien-3-on (hergestellt nach 2) das entsprechende 17α-Ethinyl-16α-ethyl-17β-hydroxy-5(10),9(11)-estradien-3-on.

Anstelle mit einer metallorganischen Ethinylverbindung kann das 17-Keton auch mit anderen metallorganischen Kohlenwasserstoffverbindungen (metallorganischen $R_3$-Verbindungen) umgesetzt werden. Als metallorganische $R_3$-Verbindungen sind zum Beispiel Alkyl-, Alkenyl- und Alkinylmagnesiumhalogenide geeignet, insbesondere Methyl-, Ethyl-, Vinyl-, Ethinyl- und Propinylmagnesiumbromid oder -jodid.

Zur Chlorethinylierung kann die metallorganische Chlorethinylverbindung in situ aus 1,2-Dichlorethylen und einer etherischen Alkyllithiumlösung, wie zum Beispiel Methyl- oder Butyllithiumlösung, gebildet werden. Geeignete Lösungsmittel sind Tetrahydrofuran und Diethylether.

Wie für die Verfahrensprodukte der allgemeinen Formel I beschrieben, können auch die Ausgangsverbindungen der allgemeinen Formel II durch Verseifung der 17β-Acetoxygruppe und gegebenenfalls erneute Veresterung, Glykosidierung oder Veretherung der freien sekundären oder tertiären 17β-Hydroxygruppe in 17-Stellung abgewandelt werden.

## Beispiel 1

3,2 g 17β-Acetoxy-16α-ethyl-5(10),9(11)-estradien-3-on werden in 120 ml Toluol und 40 ml Methylenchlorid gelöst und bei Raumtemperatur tropfenweise mit einer Lösung von 5,5 g Dichlor-dicyan-p-benzochinon in 80 ml Toluol und 20 ml Methylenchlorid versetzt. Man rührt noch 16 Stunden bei Raumtemperatur, filtriert über neutrales Aluminiumoxid und engt ein. Nach Kristallisation aus Diethylether erhält man 2,8 g 17β-Acetoxy-16α-ethyl-4,9,11-estratrien-3-on vom Schmelzpunkt 95–98° C.

## Beispiel 2

Aus 3,6 g 17β-Acetoxy-16α-methyl-5(10),9(11)-estradien-3-on erhält man analog Beispiel 1 3,0 g 17β-Acetoxy-16α-methyl-4,9,11-estratrien-3-on vom Schmelzpunkt 120–123° C (aus Diethylether).

## Beispiel 3

Eine Lösung von 2,5 g 17β-Acetoxy-16α-ethyl-4,9,11-estratrien-3-on in 120 ml Methanol und 5 ml Wasser wird nach Zusatz von 6,0 g Kaliumcarbonat 4 Stunden bei Raumtemperatur gerührt. Anschließend wird in Wasser gegossen und mit Essigsäureethylester extrahiert. Nach Kristallisation des Rohprodukts aus Essigester erhält man 1,9 g 16α-Ethyl-17β-hydroxy-4,9,11-estratrien-3-on vom Schmelzpunkt 139–141° C.

## Beispiel 4

Analog Beispiel 3 werden 2,5 g 17β-Acetoxy-16α-methyl-4,9,11-estratrien-3-on verseift. Man erhält nach Kristallisation aus Acetonitril 1,95 g 17β-Hydroxy-16α-methyl-4,9,11-estratrien-3-on vom Schmelzpunkt 149–151° C.

## Beispiel 5

1,9 g 17α-Ethinyl-17β-hydroxy-16α-methyl-5(10),9(11)-estradien-3-on werden in 60 ml Benzol und 20 ml Methylenchlorid gelöst und bei Raumtemperatur tropfenweise mit einer Lösung von 3,5 g Dichlor-dicyan-p-benzochinon versetzt. Man rührt 5 Stunden bei Raumtemperatur und arbeitet wie unter Beispiel 1 auf. Nach Kristallisation aus Ether erhält man 1,65 g 17α-Ethinyl-17β-hydroxy-16α-methyl-4,9,11-estratrien-3-on vom Schmelzpunkt 176–179° C.

## Beispiel 6

Aus 3,6 g 17α-Ethinyl-16α-ethyl-17β-hydroxy-5(10),9(11)-estradien-3-on erhält man analog Beispiel 5 3,4 g 17α-Ethinyl-16α-ethyl-17β-hydroxy-4,9,11-estratrien-3-on vom Schmelzpunkt 164–166° C (Methylenchlorid/Diisopropylether).

## Beispiel 7

Aus 3,1 g 17β-Acetoxy-16α-ethyl-18-methyl-5(10),9(11)-estradien-3-on (hergestellt nach 4) erhält man analog Beispiel 1 nach Kristallisation aus Ether 2,0 g 17β-Acetoxy-16α-ethyl-18-methyl-4,9,11-estratrien-3-on vom Schmelzpunkt 123–127° C.

## Beispiel 8

Analog Beispiel 3 werden 1,5 g 17β-Acetoxy-16α-ethyl-18-methyl-4,9,11-estratrien-3-on verseift. Man erhält nach Kristallisation aus Acetonitril 1,35 g 16α-Ethyl-17β-hydroxy-18-methyl-4,9,11-estratrien-3-on vom Schmelzpunkt 184–187° C.

Beispiel 9

Eine Lösung von 2,0 g 16α-Ethyl-17β-hydroxy-4,9,11-estratrien-3-on in 30 ml absolutem Tetrahydrofuran wird mit 3 ml Dihydropyran und 0,01 ml Phosphoroxychlorid 3 Stunden bei Raumtemperatur gerührt. Anschließend gießt man in 5%ige $NaHCO_3$-Lösung und extrahiert mit Ether. Die Ether-Extrakte werden mit gesättigter NaCl-Lösung gewaschen, über $Na_2SO_4$ getrocknet und eingeengt. Es verbleibt ein hellgelbes Öl (2,2 g), das über Kieselgel mit Benzin/Essigester säulenchromatographiert wird. Kristallisation der Hauptfraktion aus Ether/Benzin ergibt 1,4 g 16α-Ethyl-17β-tetrahydropyran-2-yloxy-4,9,11-estratrien-3-on vom Schmelzpunkt 126 – 129° C (Isomerengemisch).

Beispiel 10

Eine Lösung von 1,5 g 17β-Acetoxy-16α-n-propyl-5(10),9(11)-estradien-3-on in 100 ml Methylenchlorid wird mit 1,8 g Dichlor-dicyan-p-benzochinon unter den Bedingungen des Beispiels 1 dehydriert. Man erhält nach Kristallisation aus Diisopropylether 1,4 g 17β-Acetoxy-16α-n-propyl-4,9,11-estratrien-3-on vom Schmelzpunkt 108 – 109° C.

Beispiel 11

Aus 1,2 g 17β-Acetoxy-16α-n-propyl-4,9,11-estratrien-3-on erhält man durch Verseifung analog Beispiel 3 1,0 g 17β-Hydroxy-16α-n-propyl-4,9,11-estratrien-3-on vom Schmelzpunkt 135 – 136° C (Diisopropylether).

**Patentansprüche für die Vertragsstaaten: BE, CH, DE, FR, GB, IT, LI, LU, NL, SE**

1. 16α-Alkylsteroide der allgemeinen Formel I

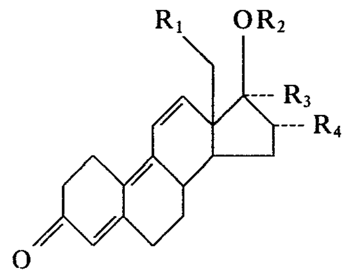

(I)

worin

$R_1$ ein Wasserstoffatom oder eine Methylgruppe,
$R_2$ ein Wasserstoffatom, eine Alkyl-, Acyl- oder Glykosylgruppe,
$R_3$ ein Wasserstoffatom oder einen substituierten oder unsubstituierten, gesättigten oder ungesättigten niederen Kohlenwasserstoffrest und
$R_4$ eine Methyl-, Ethyl- oder Propylgruppe

bedeuten.

2. 17β-Acetoxy-16α-ethyl-4,9,11-estratrien-3-on.
3. 16α-Ethyl-17β-hydroxy-4,9,11-estratrien-3-on.
4. 17β-Acetoxy-16α-methyl-4,9,11-estratrien-3-on.
5. 17β-Hydroxy-16α-methyl-4,9,11-estratrien-3-on.
6. 17β-Acetoxy-16α-propyl-4,9,11-estratrien-3-on.
7. 17β-Hydroxy-16α-propyl-4,9,11-estratrien-3-on.
8. 17α-Ethinyl-17β-hydroxy-16α-methyl-4,9,11-estratrien-3-on.
9. 17α-Ethinyl-16α-ethyl-17β-hydroxy-4,9,11-estratrien-3-on.
10. 17β-Acetoxy-16α-ethyl-18-methyl-4,9,11-estratrien-3-on.
11. 16α-Ethyl-17β-hydroxy-18-methyl-4,9,11-estratrien-3-on.
12. 16α-Ethyl-17β-tetrahydropyran-2-yloxy-4,9,11-estratrien-3-on.
13. Pharmazeutische Präparate, enthaltend eine Verbindung gemäß Ansprüche 1 bis 11.

14. Verfahren zur Herstellung von 16α-Alkylsteroiden der allgemeinen Formel I gemäß Anspruch 1, dadurch gekennzeichnet, daß man ein 16α-Alkylsteroid der allgemeinen Formel II

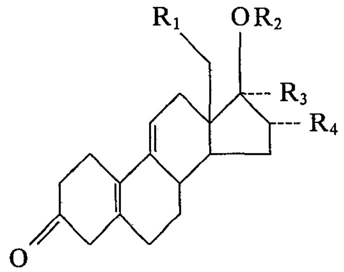

(II)

in der $R_1$, $R_2$, $R_3$ und $R_4$ die angegebene Bedeutung haben, dehydriert und gegebenenfalls anschließend eine veresterte sekundäre 17β-Hydroxygruppe verseift und/oder eine freie sekundäre oder tertiäre 17β-Hydroxygruppe verestert, glykosidiert oder veräthert.

**Patentanspruch für den Vertragsstaat: AT**

Verfahren zur Herstellung von 16α-Alkylsteroiden der allgemeinen Formel I

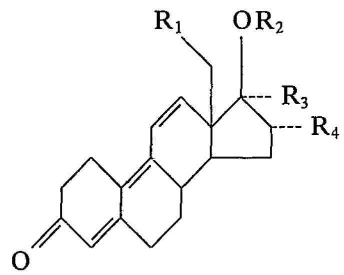

(I)

worin

$R_1$ ein Wasserstoffatom oder eine Methylgruppe,
$R_2$ ein Wasserstoffatom, eine Alkyl-, Acyl- oder Glykosylgruppe,
$R_3$ ein Wasserstoffatom oder einen substituierten oder unsubstituierten, gesättigten oder ungesättigten niederen Kohlenwasserstoffrest und
$R_4$ eine Methyl-, Ethyl- oder Propylgruppe

bedeuten,
dadurch gekennzeichnet, daß man ein 16α-Alkylsteroid der allgemeinen Formel II

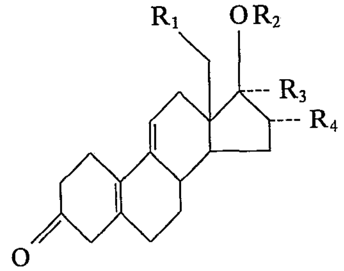

(II)

in der $R_1$, $R_2$, $R_3$ und $R_4$ die angegebene Bedeutung haben, dehydriert und gegebenenfalls anschließend eine veresterte sekundäre 17β-Hydroxygruppe verseift und/oder eine freie sekundäre oder tertiäre 17β-Hydroxygruppe verestert, glykosidiert oder veräthert.

**Claims for the Contracting States: BE, CH, DE, FR, GB, IT, LI, LU, NL, SE**

1. 16α-alkyl steroids of the general formula I

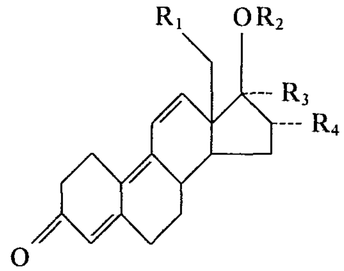

(I)

in which

$R_1$ denotes a hydrogen atom or a methyl group,
$R_2$ denotes a hydrogen atom, an alkyl, acyl or glycosyl group,
$R_3$ denotes a hydrogen atom or a substituted or unsubstituted, saturated or unsaturated lower hydrocarbon radical and
$R_4$ denotes a methyl, ethyl or propyl group.

2. 17β-acetoxy-16α-ethyl-4,9,11-oestratrien-3-one.
3. 16α-ethyl-17β-hydroxy-4,9,11-oestratrien-3-one.
4. 17β-acetoxy-16α-methyl-4,9,11-oestratrien-3-one.
5. 17β-hydroxy-16α-methyl-4,9,11-oestratrien-3-one.
6. 17β-acetoxy-16α-propyl-4,9,11-oestratrien-3-one.
7. 17β-hydroxy-16α-propyl-4,9,11-oestratrien-3-one.
8. 17α-ethynyl-17β-hydroxy-16α-methyl-4,9,11-oestratrien-3-one.
9. 17α-ethynyl-16α-ethyl-17β-hydroxy-4,9,11-oestratrien-3-one.
10. 17β-acetoxy-16α-ethyl-18-methyl-4,9,11-oestratrien-3-one.
11. 16α-ethyl-17β-hydroxy-18-methyl-4,9,11-oestratrien-3-one.
12. 16α-ethyl-17β-tetrahydropyran-2-yloxy-4,9,11-oestratrien-3-one.
13. Pharmaceutical preparations containing a compound according to claims 1 to 11.
14. Process for the manufacture of 16α-alkyl steroids of the general formula I according to claim 1, characterised in that a 16α-alkyl steroid of the general formula II,

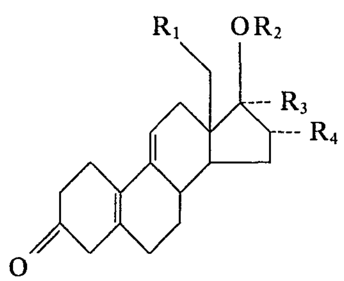

(II)

in which $R_1$, $R_2$, $R_3$ and $R_4$ have the meanings indicated, is dehydrogenated and optionally an esterified secondary 17β-hydroxy group is then hydrolysed and/or a free, secondary or tertiary 17β-hydroxy group is esterified, glycosidated or etherified.

**Claim for the Contracting State: AT**

Process for the manufacture of 16α-alkyl steroids of the general formula I

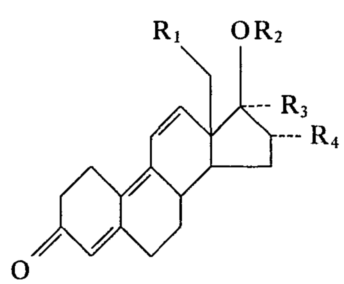

(I)

in which

$R_1$ denotes a hydrogen atom or a methyl group,
$R_2$ denotes a hydrogen atom, an alkyl, acyl or glycosyl group,
$R_3$ denotes a hydrogen atom or a substituted or unsubstituted, saturated or unsaturated lower hydrocarbon radical and
$R_4$ denotes a methyl, ethyl or propyl group,

characterised in that a 16α-alkyl steroid of the general formula II

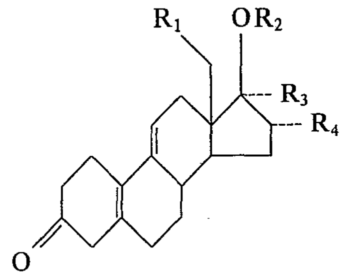

(II)

in which $R_1$, $R_2$, $R_3$ and $R_4$ have the meanings indicated, is dehydrogenated and optionally an esterified secondary 17β-hydroxy group is then hydrolysed and/or a free, secondary or tertiary 17β-hydroxy group is esterified, glycosidated or etherified.

**Revendications pour les Etats contractants: BE, CH, DE, FR, GB, IT, LI, LU, NL, SE**

1. Alkyl-16α stéorïdes répondant à la formule générale I

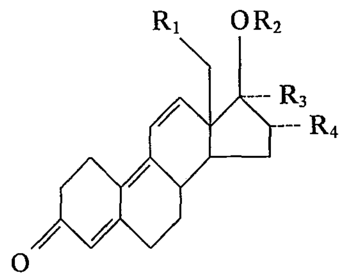

(I)

dans laquelle:

$R_1$ représente un atome d'hydrogène ou un radical méthyle,
$R_2$ représente un atome d'hydrogène ou un radical alkyle, acyle ou glycosyle,
$R_3$ représente un atome d'hydrogène ou un radical hydrocarboné inférieur, substitué ou non, saturé ou non, et
$R_4$ représente un radical méthyle, éthyle ou propyle.

2. Acétoxy-17β éthyl-16α œstratriène-4,9,11 one-3.
3. Ethyl-16α hydroxy-17β œstratriène-4,9,11 one-3.
4. Acétoxy-17β méthyl-16α œstratriène-4,9,11 one-3.
5. Hydroxy-17β méthyl-16α œstratriène-4,9,11 one-3.
6. Acétoxy-17β propyl-16α œstratriène-4,9,11 one-3.
7. Hydroxy-17β propyl-16α œstratriène-4,9,11 one-3.
8. Ethynyl-17α hydroxy-17β méthyl-16α œstratriène-4,9,11 one-3.
9. Ethynyl-17α éthyl-16α hydroxy-17β œstratriène-4,9,11 one-3.
10. Acétoxy-17β éthyl-16α méthyl-18 œstratriène-4,9,11 one-3.
11. Ethyl-16α hydroxy-17β méthyl-18 œstratriène-4,9,11 one-3.
12. Ethyl-16α (tétrahydropyrannyl-2 oxy)-17β œstratriène-4,9,11 one-3.
13. Médicaments contenant un composé selon l'une quelconque des revendications 1 à 11.

14. Procédé de préparation d'alkyl-16α stéroïdes de formule générale I selon la revendication 1, procédé caractérisé en ce qu'on déshydrogène un alkyl-16α stéroïde répondant à la formule générale II

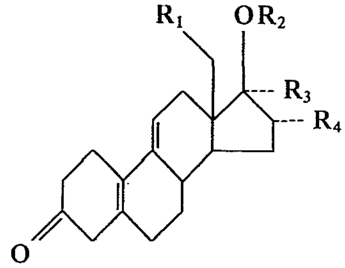

(II)

dans laquelle, $R_1$, $R_2$, $R_3$ et $R_4$ ont les significations déjà données, puis, le cas échéant, on saponifie un groupe hydroxy-17β secondaire estérifié et/ou on estérifie, glycosyle ou éthérifie un groupe hydroxy-17β, secondaire ou tertiaire, libre.

**Revendication pour l'Etat contractant: AT**

Procédé de préparation d'alkyl-16α stéroides de formule générale I

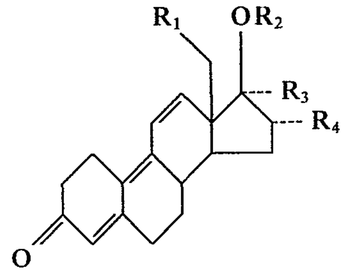

(I)

dans laquelle:

$R_1$ représente un atome d'hydrogène ou un radical méthyle,
$R_2$ représente un atome d'hydrogène ou un radical alkyle, acyle ou glycosyle,
$R_3$ représente un atome d'hydrogène ou un radical hydrocarboné inférieur, substitué ou non, saturé ou non, et
$R_4$ représente un radical méthyle, éthyle ou propyle

procédé caractérisé en ce qu'on déshydrogène un alkyl-16α stéroide répondant à la formule générale II

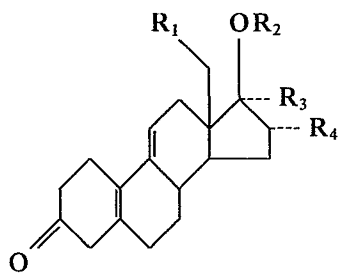

(II)

dans laquelle $R_1$, $R_2$, $R_3$ et $R_4$ ont les significations déjà données, puis, le cas échéant, on saponifie un groupe hydroxy-17β secondaire estérifié et/ou on estérifie, glycosyle ou éthérifie un groupe hydroxy-17β, secondaire ou tertiaire, libre.